# EUROPEAN PATENT APPLICATION

(11) **EP 4 115 898 A1**
(43) Date of publication of application: **11.01.2023**
(21) Application number: 20887502.1
(22) Date of filing: 09.11.2020
(51) Int. Cl.: A61K 38/18, A61P 25/28

(54) **USE OF GROUP OF SNAKE NERVE GROWTH FACTORS AND SNAKE NERVE GROWTH FACTOR PRECURSORS IN TREATMENT OF SENILE DEMENTIA**

(30) Priority: 16.11.2019 CN 201911146260
(71) Applicant: Qi, Zhankai, Shanghai 200065 (CN)
(72) Inventor: QI, Hyatt, Shanghai 200065 (CN); QI, Zhankai, Shanghai 200065 (CN)
(74) Representative: Sun, Yiming
(86) International application number: PCT/CN2020/000270
(87) International publication number: WO 2021/093134

(57) **Abstract**

A pharmaceutical composition for treating senile dementia. The composition contains a therapeutically effective amount of nerve growth factors or nerve growth factor precursors of Agkistrodon or viper of the viperidae family, and a pharmaceutically acceptable carrier base for use in reversing or relieving the symptoms of senile dementia.

## Description

### TECHNICAL FIELD

The invention relates to a group of nerve growth factor precursor and nerve growth factor of viperidae family that can improve the learning and memory ability of Alzheimer's Disease rats, and a pharmaceutical composition and method for treating Alzheimer's Disease, belonging to the field of biopharmaceutical area.

### BACKGROUND OF TECHNOLOGY

Senile dementia, also known as Alzheimer's disease (AD), is a senile disease characterized by progressive memory impairment, thinking and reasoning ability impairment, and movement disorder. Dementia is an acquired and persistent mental retardation syndrome caused by brain dysfunction, the incidence rate and prevalence of dementia are increasing with age. With the serious problem of the increase of aging population, AD has become the fourth leading cause of death.

The pathogenesis of Alzheimer's disease is the degenerative lesion of the nervous system with brain cortical diffused atrophy accompanied by neuronal damage and death. Therefore, promoting the growth and repair of neurons is a directed approach for the treatment of Alzheimer's disease.

Through animal experiments and clinical observation, the researchers found that nerve growth factor (NGF) can have the following effects on the nervous system:
(1) Protection of sensory neurons: NGF can protect sensory neurons from degeneration and death after nerve injury, preserve the essential material for nerve fiber regeneration and improve the nerve recovery rate.
(2) Promote the regeneration of motor fibers: at present, it is proved that nerve growth factor (NGF) can not only directly act on motor neurons, but also promote the synthesis of ornithine metabolite polyhydroxyamine after nerve damage, so as to promote the extension of motor axons and protect the survival of neurons.

For the treatment of Alzheimer's disease, although there are reports that snake venom is used to treat Alzheimer's disease, however there are different kinds of snake venom. So far, there is no report on nerve growth factor monomer or its precursor of viperidae family with the determined amino acid sequence that is effective for the treatment of Alzheimer's disease. The invention disclose for the first time a group of nerve growth factor monomers of viperidae family with the determined amino acid sequence and their precursors to improve the cognitive ability of senile dementia rats. At the same time, the invention disclose for the first time that nerve growth factor precursors may have a better therapeutic effect on senile dementia rats than nerve growth factors through animal models.

Many prokaryotic and eukaryotic proteins are synthesized in the form of precursors with prepro-peptides. Prepro-peptides play an important role in helping the correct folding of proteins and the correct pairing of disulfide bonds. In cells, snake nerve growth factor is composed of 200 ~ 300 amino acids, commonly known as nerve growth factor precursor (prepro-NGF). Nerve growth factor precursor (prepro NGF) is the initial form of nerve growth factor in animals. It is a precursor protein composed of amino acid signal peptide (pre peptide) + amino acid precursor peptide (pro peptide) + amino acid mature peptide. The pro- peptide, can catalyze the correct folding and processing of proteins. This pro-peptides is called intra-molecular chaperone (IMC) [1].

In 1978, Laskey et al. first time indicated the concept of molecular chaperone [2], and then successively found proteins with similar functions in prokaryotes and eukaryotes. The common feature is that they can participate in catalyzing and mediating the folding and assembly of other polypeptide chains or oligomeric protein molecules.

In 1987, Ikemura found that the post-translational folding of subtilisin needs the help of Pro peptide [3]. Subsequent studies have shown that in addition to Bacillus subtilis, many proteins synthesized in the form of precursors cannot fold correctly in the absence of their prepro- peptides, such as a-hydrolytic protease (ALP), carboxypeptidase, etc. [4,5]. Therefore, it is known that the prepro-peptide plays the role of intra-molecular chaperone in the folding process of some proteins.

In 1993, shinde et al also put forward the concept of intra-molecular chaperone, and this prepro-peptide with similar functions that can assist in protein folding and processing is called intra-molecular chaperone IMC [1].

Various physical and chemical properties show that nerve growth factor is a dimer structure, which is composed of two identical peptide chains. Each peptide chain has multiple cysteine residues, forming disulfide bonds between them. These disulfide bonds are very important to maintain the biological activity of NGF. Once destroyed, the activity will be lost. In vivo, the precursor of nerve growth factor (prepro NGF) exists in the endoplasmic reticulum in the form of Pro peptide, folds into a natural dimer structure, and then is transferred to the Golgi apparatus; the prepro- peptide will be under a limitable hydrolyzation by Flynn protease and corresponding precursor protein invertase to form mature NGF dimer, which is then transported outside the cell, during this time, a few uncut Pro NGF precursors are secreted outside the cell as well. Because the pro peptide plays an important role in the folding of the functional peptide chain of nerve growth factor, that is, the formation of spatial structure [6], so while the gene recombinant technique is used for synthesis, the structure of nerve growth factor precursor (prepro NGF) with Pro peptide may be closer to the spatial structure of natural nerve growth factor dimmer than that only with nerve growth factor mature peptide. The spatial structure of the protein is the basis of biological activity, when the peptide chain is synthesized in vivo, it is folded and processed into a specific three-dimensional structure, and finally forms a bioactive protein.

More and more discoveries find that protein folding systems might have to rely on the presence of prepro- peptides [7, 8, 9, 10], which shows that this might be a universal protein folding mechanism. This phenomenon exists from prokaryotes to eukaryotes, including all kinds of proteases, such as serine, protease, cysteine protease, metalloproteinase, etc., as well as other no proteases, such as lipase, some toxin proteins, etc. The existence of prepro- peptides reduces the activation energy of folding reaction and makes the folding process easier [11]. In addition to assisting the correct folding of proteins, prepro- peptides of some proteins can also help the correct pairing of disulfide bonds [12]. As an intra-molecular chaperone (IMC), the prepro- peptide can assist protein folding, which has important meanings for guiding the recombinant protein expressed by gene engineering technology to have higher biological activity protein [13].

The nerve growth factor (NGF) of the viperidae family is a typical representative of the neurotrophic factor family. Its basic spatial structure has "cystine junction" units, and its biological active structures are non-covalently linked homodimers β Folding, so it is also called β Dimer. They also have high homology in amino acid sequence. They protect the survival, regeneration, and differentiation of neurons in the central and peripheral nervous systems. Their biological activity, structure, and function are basically the same.

### SUMMARY OF THE INVENTION

Nerve growth factor (NGF) exists in many species, and is abundant in male mouse submandibular gland, bovine seminal plasma, snake venom, guinea pig prostate and human placenta. Among them, the snake nerve growth factor (NGF) of viperidae family not only has the same spatial structure as human and mouse nerve growth factor (NGF), but also has certain homology in amino acid sequence. We have found for the first time in this invention that the nerve growth factor precursor (preproNGF) and nerve growth factor (NGF) of Agkistrodon piscivorus and Protobothrops mucrosquamatus of the viperidae family could improve the learning and memory ability of Alzheimer's rats. At the same time, we also found that the nerve growth factor precursor (preproNGF) and nerve growth factor (NGF) of the viper snake with identical spatial structure (β dimer structure) and high homology of amino acid sequence with that of Agkistrodon piscivorus and Protobothrops mucrosquamatus could also significantly improve the learning and memory ability of Alzheimer's rats, indicating that this common bioactive structure could produce the same biological activity and therapeutic effect. Further studies also found that the therapeutic effect of the nerve growth factor precursor containing prepro- peptide (preproNGF) could be better than that of nerve growth factor (NGF) because the prepro- peptide plays an important role in the folding of nerve growth factor functional peptide chain, that is, the formation of spatial structure, and the bioactivity of protein is related to the spatial structure to a large extent.

The amino acid sequences of the nerve growth factor precursor (preproNGF) and nerve growth factor (NGF) of the viperidae family Agkistrodon piscivorus, Protobothrops mucrosquamatus and viper snake used in the experiment of the invention are as follows:
Agkistrodon piscivorus
The nerve growth factor precursor (preproNGF) (SEQ ID No.1): 
msmlcytliiafligiwaapksednvplgspatsdlsdtscakthealktsrntdqhypa

pkkaedqefgsaaniivdpklfqkrrfqsprvlfstqppplsrdeqnvdnanslnrnira

kredhpvhnrgeysvcdsvnvwvankttatdirgnvvtvmdvninnnvykqyffetkcr

npnpvptgcrgidarhwnsyctttntfvkaltmegnqaswrfiridsacvcvisrknenfg
The mature peptide of nerve growth factor (SEQ ID No.2): 
hnrgeysvcdsvnvwvankttatdirgnvvtvmvdvninnnvykqyffetkcrnpnpvptgcr
gidarhwnsycttttntfvkaltmegnqaswrfiridsacvcvisrkn
The nerve growth factor precursor (preproNGF) (SEQ ID No.3): 
scmkaapmkevsirgqgslaypglrtqgnletlsgpndatrgltsladtfehvieellde

qqaiqpskenkdadlyssrvmlssqvpleppllflleeyknyldaanmsmrvrrhsdpar

rgelsvcdstsewvtaaekktavdmsgatvtvlekvpvpkgqlkqyfyetkcstkgyake

gcrgidkrywnsqcrttqsyvraltmdnkkrvgwrfiridt
The mature peptide of nerve growth factor (SEQ ID No.4): 
arrgelsvcdstsewvtaaekktavdmsgatvtvlekvpvpkgqlkqyfyetkcsakgyake

gcrgidkrywnsqcrttqsyvraltmdnkkrvgwrfiridt

Protobothrops mucrosquamatus
The nerve growth factor precursor (preproNGF) (SEQ ID No.5): 
msmlcytliiafligiwaapksednvplgspatsdlsdtscakthealktsrntdhhypa

pkkaedqefgsaaniivdpklfqkrrfqsprvlfstqppplsrdeqsvdnanslnrnira

kredhpvhnrgeysvcdsvnvwvankttatdirgnlvtvmvdvnlnnnvykqyffetkcr

npnpvptgcrgidsrhwnsycttthtfvkaltmegkqaswrfiridaacvcvisrknenfg
The mature peptide of nerve growth factor (SEQ ID No.6): 
hnrgeysvcdsvnvwvankttatdirgnlvtvmvdvnlnnnvykqyffetkcrnpnpvptgcr
gidsrhwnsycttthtfvkaltmegkqaswrfiridaacvcvisrkn

Macrovipera lebetina
The nerve growth factor precursor (preproNGF) (SEQ ID No.7): 
msmlcytliiafligiwaapksednvslgspatpdlsdtscakthealktsrntdqhypa

pkkaedqefgsaaniivdpklfqkrrfqsprvlfstqppplsrdeqsvefldnadslnrn

irakrathpvhnrgefsvcdsvsvwvankttatdirgnvvtvmvdvklnnnvyrqyffet

kcknpspvssgcrgidakhwnsyctttdtfvraltmegnqaswrfiridtacvcvisrkn

dnfg
The mature peptide of nerve growth factor (SEQ ID No.8):

hnrgefsvcdsvsvwvankttatdirgnvvtvmvdvklnnnvyrqyffetkcknpspvssgcr
gidakhwnsyctttdtfvraltmegnqaswrfiridtacvcvisrkn

The present invention is further described below with reference to the attached drawings and specific embodiments. However, the following embodiments are not intended to limit the present invention. Meanwhile, the present disclosure and the like made according to the content disclosed in the present disclosure are all common substitutions in the art and are all within the protection scope of the present invention.

### BRIEF DESCRIPTION OF THE DRAWINGS:

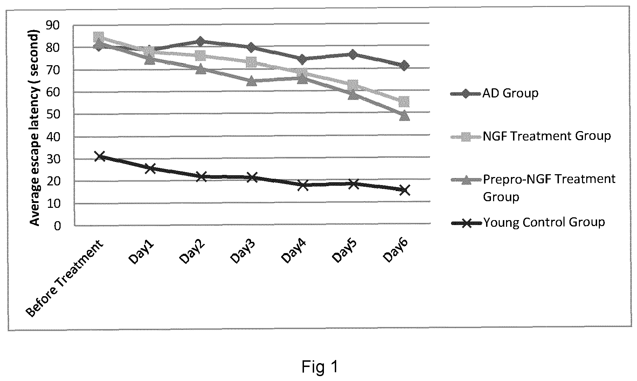

Fig 1 is a line chart corresponding to the data of table1

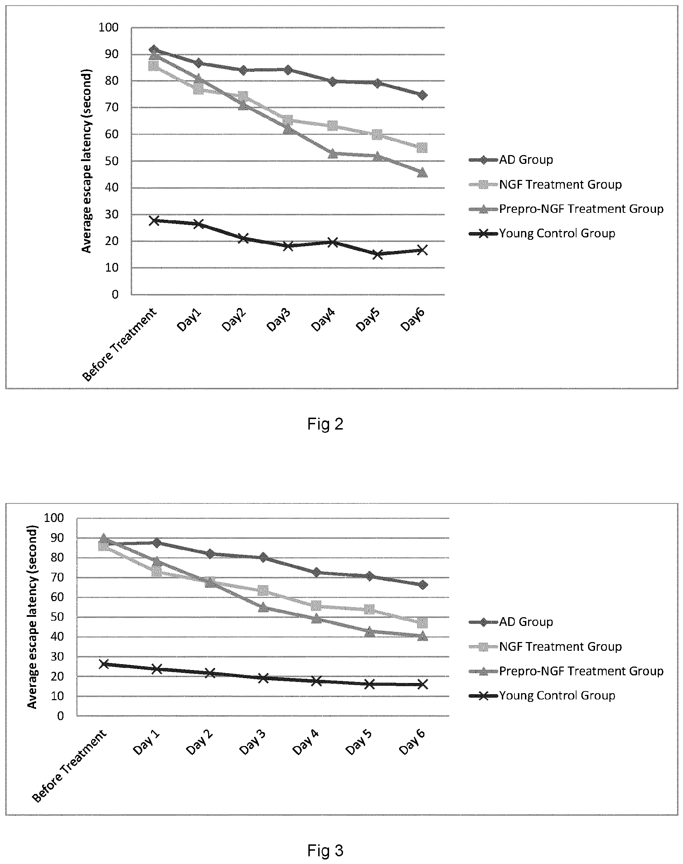

Fig 2 is a line chart corresponding to the data of table2
Fig 3 is a line chart corresponding to the data of table3

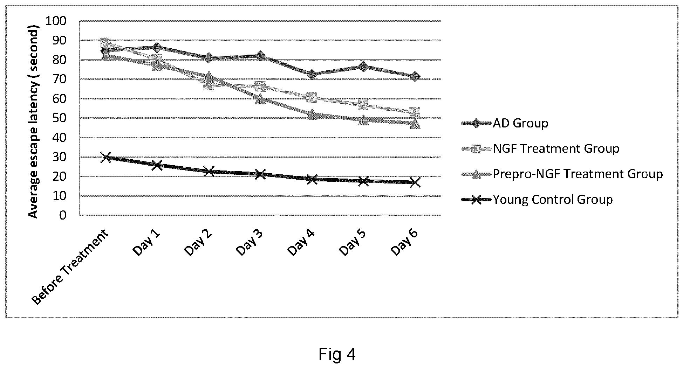

Fig 4 is a line chart corresponding to the data of table4

### METHOD OF IMPLEMENTATION

Example A: Obtaining the nerve growth factor precursor (preproNGF) and the nerve growth factor (NGF) The method comprises the following steps:
I. Agkistrodon piscivorus nerve growth factor precursor (prepro NGF) (SEQ ID No.1) was obtained by gene recombinant technology and is specified as follows. :
   1. Cloning of Recombinant Expression Vector
      A DNA sequence is synthesized according to the gene of Agkistrodon piscivorus nerve growth factor precursor (prepro NGF)
      Design and use primers Sequence(5'->3')
      Forward primer TGGGTCAGCAGCAAACATCA
      Reverse primer AGGATGCCTGATTGCCTTCC
      PCR amplification is carried out on a target DNA sequence, a sequence encoding the intestinal kinase recognition site, and an NDE I enzyme digestion site is introduced at the 5 'end of the upstream primer, and a stop codon and a BamHI enzyme digestion site are introduced at the 5 'end of the downstream primer.
      The gene containing the Agkistrodon piscivorus nerve growth factor precursor (prepro NGF)is amplified by using a PCR method and cloned into a PBS-T vector, and the constructed recombinant sub-PBS-T- Agkistrodon piscivorus nerve growth factor precursor (prepro NGF)shall be under examination of analysis and identification.
   2. Gene expression
      The recombinant plasmid is transformed into the Escherichia coli expression vector pET15b, and the recombinant expression plasmid pET15b- Agkistrodon piscivorus nerve growth factor precursor (prepro NGF) is constructed, and the correct recombinant transplanting to Escherichia coli BL21 (DE3) - LysS and examined under analysis and identification. The monoclonal antibody is inoculated into a 5ml LB culture medium, cultured overnight at 37 DEG C, the next day is inoculated into a 50 ml LB culture medium according to a ratio of 1: 100, and the culture is shaken at 37 DEG C until the OD600 nm is equal to 0.4 -0.6.
   3. Collection and Analysis of Expressed Products
      The transformation is continued with 1 mmol/L of IPTG for 3 hours, the expression bacteria, which means the induced and transformed Escherichia coli BL21 (DE3), is centrifuged, the inclusion body is dissolved in the buffer solution, and the supernatant and the precipitate are subjected to SDS-PAGE electrophoresis detection after centrifugation and collection, and the target protein exists in the form of inclusion bodies.
   4. Affinity and Purification of Expressed Products
      The inclusion body after ultrasonic breaking is dissolved in a buffer solution (6 mol/L of guanidine hydrochloride, 20 mmol/L of Tris-HCI, ph 8.0, 0.5 mol/L of Nacl, 5 mmol/L imidazole); the buffer solution is purified through a nickel-NTA column affinity chromatography, specifically, the buffer solution containing 20 mmol/L imidazole is washed to a baseline before loading, and finally, the buffer solution containing 300 mol/L imidazole is used for elution. The enterokinase is used to cut for obtaining the Agkistrodon piscivorus nerve growth factor precursor (prepro NGF).
   5. Renaturation of Expressed Product
      The eluted protein is dialyzed with 6 mol/L guanidine hydrochloride, 0.1 mol/L Tris-HCI-HCI Ph8.0, 0.01 mol LEDTA, 0.1 mmol/L PMSF, 10 mmol/L DTT buffer solution, the concentration of DTT and guanidine hydrochloride in the buffer solution is gradually decreased, and then the buffer solution is dialyzed with 10 - fold volume of 0.1 mol/L Tris-HCL Ph-8.0, 5 µmol/L CuSo4, and 20% glycerol. An RP-HPLC method is used for detecting the renaturation result, and through the comparison of retention time with a standard sample, the renaturation substance is identified, and the renaturation product is refrigerated and stored.
   6. Amino Acid Sequence Determination
      The peptide fragment coverage rate and the Edman degradation method are used for the analysis of the obtained NGF precursor, and the measured prepro NGF (SEQ ID No.1) sequence is compared with the amino acid sequence of the prepro NGF in the protein library, and after the confirmation of the consistency of the sequence, then the (prepro NGF) (SEQ ID No.1) is used for the treatment of senile dementia rat in the next step.
II. Agkistrodon piscivorus nerve growth factor ( NGF) (SEQ ID No.2) was obtained by gene recombinant technology and is specified as follows. :
   1. Cloning of Recombinant Expression Vector
      A DNA sequence is synthesized according to the gene of Agkistrodon piscivorus nerve growth factor (NGF)
      Design and use primers Sequence(5'->3')
      Forward primer ATATCCGGGCCAAACGTGAA
      Reverse primer TGATGTCCGTTGCTGTGGTT
      PCR amplification is carried out on a target DNA sequence, a sequence encoding the intestinal kinase recognition site, and an NDE I enzyme digestion site is introduced at the 5 'end of the upstream primer, and a stop codon and a BamHI enzyme digestion site are introduced at the 5 'end of the downstream primer.
      The gene containing the Agkistrodon piscivorus nerve growth factor ( NGF)is amplified by using a PCR method and cloned into a PBS-T vector, and the constructed recombinant sub-PBS-T- Agkistrodon piscivorus nerve growth factor ( NGF)shall be under examination of analysis and identification.
   2. Gene expression
      The recombinant plasmid is transformed into the Escherichia coli expression vector pET15b, and the recombinant expression plasmid pET15b- Agkistrodon piscivorus nerve growth factor ( NGF) is constructed, and the correct recombinant transplanting to Escherichia coli BL21 (DE3) - LysS and examined under analysis and identification. The monoclonal antibody is inoculated into a 5ml LB culture medium, cultured overnight at 37 DEG C, the next day is inoculated into a 50 ml LB culture medium according to a ratio of 1: 100, and the culture is shaken at 37 DEG C until the OD600 nm is equal to 0.4 -0.6.
   3. Collection and Analysis of Expressed Products
      The transformation is continued with 1 mmol/L of IPTG for 3 hours, the expression bacteria, which means the induced and transformed Escherichia coli BL21 (DE3), is centrifuged, the inclusion body is dissolved in the buffer solution, and the supernatant and the precipitate are subjected to SDS-PAGE electrophoresis detection after centrifugation and collection, and the target protein exists in the form of inclusion bodies.
   4. Affinity and Purification of Expressed Products
      The inclusion body after ultrasonic breaking is dissolved in a buffer solution (6 mol/L of guanidine hydrochloride, 20 mmol/L of Tris-HCI, ph 8.0, 0.5 mol/L of Nacl, 5 mmol/L imidazole); the buffer solution is purified through a nickel-NTA column affinity chromatography, specifically, the buffer solution containing 20 mmol/L imidazole is washed to a baseline before loading, and finally, the buffer solution containing 300 mol/L imidazole is used for elution. The enterokinase is used to cut for obtaining the Agkistrodon piscivorus nerve growth factor ( NGF).
   5. Renaturation of Expressed Product
      The eluted protein is dialyzed with 6 mol/L guanidine hydrochloride, 0.1 mol/L Tris-HCI-HCI Ph8.0, 0.01 mol LEDTA, 0.1 mmol/L PMSF, 10 mmol/L DTT buffer solution, the concentration of DTT and guanidine hydrochloride in the buffer solution is gradually decreased, and then the buffer solution is dialyzed with 10 - fold volume of 0.1 mol/L Tris-HCL Ph-8.0, 5 µmol/L CuSo4, and 20% glycerol. An RP-HPLC method is used for detecting the renaturation result, and through the comparison of retention time with a standard sample, the renaturation substance is identified, and the renaturation product is refrigerated and stored.
   6. Amino Acid Sequence Determination
      The peptide fragment coverage rate and the Edman degradation method are used for the analysis of the obtained NGF, and the measured NGF (SEQ ID No.2) sequence is compared with the amino acid sequence of the NGF in the protein library, and after the confirmation of the consistency of the sequence, then the ( NGF) (SEQ ID No.2) is used for the treatment of senile dementia rat in the next step.
III. Other snake nerve growth factor precursors (preproNGF) and nerve growth factor (NGF) (SEQ ID No.3-seq ID No.8) were obtained according to the genes of the above nerve growth factor precursors (preproNGF) or nerve growth factor (NGF) mature peptides provided on Gen Bank by using the same recombinant technology, based on method I or II, other target products (SEQ ID No.3-seq ID No.8) described in the invention can be obtained and stored in cold storage for testing the animal model of Alzheimer's disease in rats.

### B. Selection of Animal Model

According to the invention, a natural aging cognitive disorder (senile dementia) animal model is adopted, animal models of senile dementia are obtained through natural aging of animals, such as aged rats and the like, and nervous system changes such as cognitive impairment of the model are naturally occurring, and are closer to the real pathological changes of AD. Cummings reported the Aβ precipitated plaque in the brain of aged canine accompanied by corresponding selective behavior impairment; Aβ deposited in the forebrain substrate area was also reported by Higgins et al., and a memory defect was detected. In our embodiment, we have used a natural aging cognitive disorder (senile dementia) animal model.

### C. Animals and Grouping

1. 200 rats with age between 21 -22 months are adaptively fed for 10 days, the animals are free to take water, the room temperature is controlled to be 22 -25 DEG C, the humidity is 50 -70%, the illumination is 12 hours, and the dark is 12 hours.
2. Rats (senile dementia rats) of cognitive disorders are screened through Morris water maze experiment training. The Morris water maze experiment is an experiment for forcing animals to swim and searching for an underwater platform, is mainly used for evaluating the animal space learning memory ability, is sensitive and reliable in experiment index, is simple and convenient to operate, and is a classical experiment for testing and evaluating senile dementia indexes of rats.
3. The water maze water depth is 50 cm, the water temperature is controlled at 22 -25 DEG C, and a platform is arranged in the pool center. The milk powder is put into the water tank and fully mixed until the water is milky white so that the rat cannot visually identify the position of the platform. During training days before formal testing, the rat, with the position facing to a wall, is put into water to find an evasion platform, if the rat finds the platform and stands for 3 seconds on the platform without slipping off, the training can be terminated, the time for reaching the platform and the distance are recorded, and the rat is allowed to stay for 10 seconds on the platform, thereby allowing the rat to learn memory. Those who can't find the platform continue to be recorded for 120 s, then guided to the platform and placed for 30 seconds, and the learning memory is trained. Training is carried out 4 times every day, the intervals between each training are about 15 -30 seconds, and rats get into water from four different directions, such training last for 4 days. The day 5th is the formal test day, the real-time image system automatically records the activity of the rat, and calculates the average time of the rat's first crossing platform (central region) to evaluate the learning memory ability of the animal. The upper limit value of 99% of the normal range of the average evasion latency of the rat (4 months old) is taken as a standard, and the old rat with an escape latency greater than 99% of the upper limit value is considered to be a known cognitive disorder old rat. Each rat is labeled so that the average evasion latency before treatment of each group of rats can be calculated after the completion of the last test.
4. The rats with cognitive disorder are screening out and randomly divided into two parts, and a part of the rats is randomly divided into three groups : ie, normal saline as senile dementia rat control group (non-drug administration, same volume saline for intragastric administration) and two snakes nerve growth factor and snakes nerve growth factor precursor as treatment group (the snakes nerve growth factor and nerve growth factor precursor is prepared into a liquid form according to 40 µg/kg, continuously intra-gastric administration, 2 times per day, and 8 weeks of continuous administration), and meanwhile, a young rat 'control group (no drug, intragastric administration of same volume of physiological saline) is set up, so that ten rats are kept for each group, and redundant rats are out of experimental group. Since there were four kinds of snake nerve growth factor and nerve growth factor precursors, so there were total 16 groups.

### D. Behavior Test (Learning and Memory ability)

After 8 weeks of treatment followed by continuous training for 5 days, during which every day's average time of all groups of rats for finding a platform in water was recorded, and Morris water maze test is directly carried out on day 6. The time for finding a platform in water for each group of rats is tested. The treatment agent is still continuously administered during the training periods.

### E. Experimental Results

Below, taking four kinds of snakes nerve growth factor and nerve growth factor precursors as examples, the experimental results of average escape latency of Alzheimer's rat control group, nerve growth factor group, nerve growth factor precursor group and young rat control group are compared to illustrate the effect of snake nerve growth factor and nerve growth factor precursors described in this disclosure on the improvement of cognitive ability of Alzheimer's rats. The results are shown in table 1-4:

**Table 1 Agkistrodon piscivorus nerve growth factor precursors( prepro NGF)(SEQ ID No.1)and nerve growth factor (NGF)(SEQ ID No.2)were used.**

| (Second)X±S, n = 10 | Before Treatment | Day 1 | Day 2 | Day 3 | Day 4 | Day 5 | Day 6 |
|---|---|---|---|---|---|---|---|
| AD Group | 81 ±9.1 | 79 ±11.7 | 82 ±10.5 | 80 ±13.9 | 74 ±11.2 | 76 ±13.8 | 71 ±16.2 |
| NGF Treatment Group | 85 ±11.5 | 78 ±13.5 | 76 ±17.3 | 73 ±13.5 | 68 ±11.9 | 62 ±11 ^{∗} | 55 ±8.6^{∗} |
| Prepro-NGF Treatment Group | 82 ±14.1 | 75 ±9.8 | 70 ±9.6 # | 65 ±11.6 # | 66 ±9.9 | 58 ±7.8 ## | 49 ±10.1## |
| Young Control Group | 31 ±6.1 | 26 ±6.1 | 22 ±3.6 | 21 ±8.1 | 18 ±2.4 | 18 ±6.5 | 15 ±3.1 |

**Table 2 Agkistrodon piscivorus nerve growth factor precursors( prepro NGF)(SEQ ID No.3)and nerve growth factor (NGF)(SEQ ID No.4) were used.**

| (Second)X±S, n = 10 | Before Treatment | Day 1 | Day 2 | Day 3 | Day 4 | Day 5 | Day 6 |
|---|---|---|---|---|---|---|---|
| AD Group | 92 ±9.8 | 87 ±11.6 | 84 ±6.3 | 84 ±7.5 | 80 ±7.6 | 79 ±11.5 | 75 ±9.9 |
| NGF Treatment Group | 86 ±9.3 | 77 ±7.0 ^{∗} | 74 ±8.3^{∗∗} | 65 ±6.4 ^{∗∗∗} | 63 ±11.8^{∗∗} | 60 ±10.5^{∗∗∗} | 55 ±8.5 ^{∗∗∗} |
| Prepro-NGF Treatment Group | 90 ±8.1 | 81 ±8.4 | 71 ±11## | 62 ±11.6### | 53 ±7.4### | 52 ±9.2### | 46 ±7.6### |
| Young Control Group | 28 ±5 | 27 ±5.7 | 21 ±3.3 | 18 ±3.9 | 20 ±5.8 | 15 ±2.7 | 17 ±3.2 |

**Table 3 Protobothrops mucrosquamatus nerve growth factor precursors( prepro NGF)(SEQ ID No.5)and nerve growth factor (NGF)(SEQ ID No.6)were used.**

| (Second)X±S, n = 10 | Before Treatment | Day 1 | Day 2 | Day 3 | Day 4 | Day 5 | Day 6 |
|---|---|---|---|---|---|---|---|
| AD Group | 87 ±5.3 | 88 ±9.9 | 82 ±5.3 | 80 ±8.0 | 73 ±8.9 | 71 ±9.5 | 66 ±6.0 |
| NGF Treatment Group | 86 ±8.1 | 73 ±8.6 ^{∗∗} | 68 ±6.6^{∗∗∗} | 63 ±6.9 ^{∗∗∗} | 56 ±5.9 ^{∗∗∗} | 54 ±7.1 ^{∗∗∗} | 47 ±6.9^{∗∗∗} |
| Prepro-NGF Treatment Group | 90 ±7.1 | 78 ±9.9 # | 68 ±6.6### | 55 ±7.4 ### | 49 ±5.0### | 43 ±7.0 ### | 41 ±5.6 ### |
| Young Control Group | 26 ±4.6 | 24 ±5.3 | 22 ±3.6 | 19 ±6.3 | 18 ±2.3 | 16 ±2.8 | 16 ±3.4 |

**Table 4 Macrovipera lebetina nerve growth factor precursors( prepro NGF)(SEQ ID No.7)and nerve growth factor (NGF)(SEQ ID No.8)were used .**

| (Second)X±S, n = 10 | Before Treatment | Day 1 | Day 2 | Day 3 | Day 4 | Day 5 | Day 6 |
|---|---|---|---|---|---|---|---|
| AD Group | 85 ±8.4 | 87 ±9.1 | 81 ±7.1 | 82 ±7.4 | 73 ±8.9 | 77 ±10.6 | 72 ±7.7 |
| NGF Treatment Group | 89 ±8.5 | 80 ±9.1 | 67 ±8.6^{∗∗∗} | 66 ±8.1^{∗∗∗} | 61 ±8.7^{∗∗} | 57 ±8.3^{∗∗∗} | 53 ±9.7 ^{∗∗∗} |
| Prepro-NGF Treatment Group | 82 ±7.8 | 77 ±10.1# | 72 ±7.4## | 60 ±8.7### | 52 ±9.9### | 49 ±10.4### | 47 ±6.8### |
| Young Control Group | 30 ±4.7 | 26 ±5.8 | 23 ±2.9 | 21 ±6.3 | 19 ±2.8 | 18 ±3.1 | 17 ±3.5 |

The above experimental data showed that there was no significant difference between the AD Group, the nerve growth factor group and the nerve growth factor precursor group before treatment. After 8 weeks of treatment, Morris water maze positioning experiment training was conducted again. From the first day to the fifth day, significant differences began to appear between the control group of Alzheimer's rats, the nerve growth factor treatment group, and nerve growth factor precursor treatment group. By the sixth day, there were significant differences between the control group of Alzheimer's rats, the nerve growth factor treatment group, and nerve growth factor precursor treatment group. The AD control group, when compared with the nerve growth factor treatment group, * means P < 0.05, * * means P < 0.01, * * * means P < 0.001; when compared with the NGF precursor group, # means P < 0.05, ## means P < 0.01, ### means P < 0.001.

The above data show that NGF precursor treatment group and NGF treatment group can shorten the average escape latency and improve the learning and memory ability of Alzheimer's rats; at the same time, nerve growth factor precursor group may have better curative effect than nerve growth factor group.

The above embodiments are only illustrative of the embodiments and technical features of the present invention, and the purpose of the present invention is to enable the skilled person in the art to understand the contents of the present invention but not to be construed as limiting the scope of protection of the present invention. Any equivalent change or modification made according to the spirit of the present invention shall fall within the protection scope of the present invention.

Ref:
1. Shinde U, Inouye M. Intramolecular chaperones: polypeptide extensions that modulate protein folding. Cell Dev Biol, 2000. II: 35-34
2. BK Choi, P Bobrowicz, RC Davidson, ST Hamilton, DH Kung, et a1. Use of combinatorial genetic libraries to humanize N-linked glycosylation in the yeast Pichia pastoris. PNAS, 2003, 1 00(9): 5022 5027
3. Ikemura H, Takagi H, Inouye M [ ] I Biol Chem, I987, 262(16): 7859-7864
4. Silen, Agard DA. Nature, 1989, 341(6241): 462 464
5. Winther JR, Sorensen P. Proc Natl Acad Sci USA, 1991,88: 9330~9334
6. Alike Rattenholl et a1. Pro-sequence assisted folding and disulfide bond formation of human nerve growth factor. J Mol Biol, 200 1, 305(3): 523-533
7. Shinde U, Inouye M. Intramolecular chaperones and protein folding. Trends Biochem Sci, 1993, 18: 442-446
8. Andemson E, Hellman L, Gullberg U, Olsson I. The role of the propeptide for processing and sorting of human myeloperoxidase [J]. Biol Chem, 1998, 273: 4747 4753
9. Shinde U, Fu X, Inouye M. A pathway for conformational diversity in proteins mediated by intramolecular chaperones [J]. Biol Chem, I999, 274: I56I5~I5621
10. Yukihiro Yabuta, Ezhilkani Subbian, et al, Folding Pathway Mediated by an Intramolecular Chaperone: A functional peptide chaperone designed using sequence databases[J]. Biol and Chem, 2003, 278(17): 15246∼I525I
11. Erin L. Cunningham , Sheila S. Jaswal , Julie L. Sohl, andDavid A. Agard. Kinetic stability as a mechanism for protease longevity. PNAS, Sep, 1999, 96: 11008 - 11014
12. Y Hidaka , M Ohno, B Hemmasi , O Hill, WG Forssmann , and Y Shimonishi. In vitro disulfide-coupled tblding of guanylyl cyclase-activating peptide and its precursor protein. Biochemistry, June 9, I998, 37(23): 8498-8507
13. Zhu Min, intramolecular chaperone and its mechanism of protein folding Chinese Journal of modern practical medicine. February 2006, Vol. 5, No. 12

## Claims

1. A method for treating senile dementia in a mammal. Said method comprising administering to a mammal in need thereof a pharmaceutical composition of a therapeutically effective amount of nerve growth factor precursor or nerve growth factor of viperidae family, and a pharmaceutically acceptable carrier base for use in reversing or relieving the symptoms of senile dementia.

2. The nerve growth factor precursor or nerve growth factor of viperidae family of claims (1), wherein it is an nerve growth factor precursor or nerve growth factor of viperidae family having the amino acid sequence shown in SEQ ID No.1 to SEQ ID No.8; or nerve growth factor precursor or nerve growth factor of viperidae family homologues having 90% or more homology with the nerve growth factor precursor or nerve growth factor of viperidae family of SEQ ID No.1 to SEQ ID No.8, and the biological function of the nerve growth factor precursor or nerve growth factor of viperidae family homologues is the same as or similar to that of the nerve growth factor precursor or nerve growth factor of viperidae family of the amino acid sequence ID No. 1 to SEQ ID No. 8.

3. The nerve growth factor precursor and nerve growth factor of viperidae family or nerve growth factor precursor and nerve growth factor of viperidae family homologues according to claims (1-2), are further **characterized in that** they are derived from natural snake venoms, or synthesized from chemical polypeptides, or obtained from prokaryotic or eukaryotic hosts using recombinant technology ( such as Bacteria, yeast, higher plants, insects and mammalian cells).

4. The recombinantly produced nerve growth factor precursor and nerve growth factor of viperidae family or its homologues according to claim (1-3), based on the host used in the recombinant production scheme, the polypeptide or its homologues of the present invention may be glycosylated, or may be non-glycosylated; Disulfide-bonded or non-disulfide-bonded. The proteins and its homologues described in the present invention may also include or exclude the starting methionine residue.

5. The nerve growth factor precursor and nerve growth factor of viperidae family as in any of claims (1-4), further **characterized in that** its protein in the present invention may include fragments of the above-mentioned nerve growth factor precursor or nerve growth factor of viperidae family protein after hydrolysis or enzymolysis, derivatives or analogs treated by physical, chemical or biological method, they are proteins which basically maintain the same biological function or activity as the above-mentioned nerve growth factor precursor or nerve growth factor of viperidae family. The fragments, derivatives or analogs described in the present invention may be a protein in which one or more amino acid residues are substituted, or a protein having a substituent group in one or more amino acid residues, or combined with a compound (such as compounds that extend the half-life of a polypeptide, such as polyethylene glycol), or a protein formed by fusion of a fatty chain, or a protein formed by fusing an additional amino acid sequence to this polypeptide sequence. As described herein, these fragments, derivatives, and analogs are within the scope of those skilled in the art.

6. The method of claims (1) comprising intravenous, intramuscular, subcutaneous, intra-articular, oral, sublingual, nasal, rectal, topical, intradermal, intraperitoneal, intrathecal administration or transdermal administration.

7. The dose of nerve growth factor precursor or nerve growth factor of viperidae family of the method of claim (1) includes from 1 µg / Kg to 500 µg / kg each time, and the injection frequency ranges from once a day to multiple times a day, or multiple times a year.
